# EUROPÄISCHE PATENTSCHRIFT

(11) **EP 1 386 648 B1**
(45) Veröffentlichungstag und Bekanntmachung des Hinweises auf die Patenterteilung: **30.11.2005**
(21) Anmeldenummer: 03015957.8
(22) Anmeldetag: 14.07.2003
(51) Int. Cl.: B01D 3/00, B01D 3/16, B01J 19/32

(54) **Verfahren zur Durchführung einer heterogen katalysierten Reaktivdestillation in Suspensionsfahrweise**
Heterogeneously catalysed reactive distillation process in suspension mode
Méthode de distillation réactive à catalyse hétérogène en mode de suspension

(30) Priorität: 03.08.2002 DE 10235552
(43) Veröffentlichungstag der Anmeldung: 04.02.2004
(73) Patentinhaber: BASF Aktiengesellschaft, 67056 Ludwigshafen (DE)
(72) Erfinder: Miller, Christian, 67152 Ruppertsberg (DE); Kaibel, Gerd, Dr., 68623 Lampertheim (DE); Siegel, Hardo, Dr., 67346 Speyer (DE)

(56) Entgegenhaltungen:
- US-A- 4 187 169
- US-A- 4 443 559
- US-A- 5 498 318
- US-A- 5 510 089
- US-A- 5 856 606

## Beschreibung

Die vorliegende Erfindung betrifft ein verbessertes Verfahren zur Durchführung einer Reaktivdestillation in Gegenwart eines heterogenen Katalysators, der als disperse Phase in der Flüssigkeit suspendiert ist.

Für die Durchführung von heterogen katalysierten Reaktivdestillationen haben sich in der Technik verschiedene Methoden etabliert.

Eine Möglichkeit besteht darin, die aktive Katalysatormasse direkt auf die Packung aufzubringen. Die hierbei verwendeten Pakkungen entsprechen den aus der Destillationstechnik allgemein gebräuchlichen Bauformen. Als Beispiel kann hier die Bauform KATA-PAK-M® der Firma Sulzer AG genannt werden. Problematisch für den technischen Einsatz ist allerdings, dass sich viele katalytisch aktiven Massen nicht mit der erforderlichen Abriebfestigkeit auf solche Packungen aufbringen lassen. Dies führt zu einer Verschlechterung der Wirtschaftlichkeit dieser Verfahren und erhöht gleichzeitig den benötigten verfahrenstechnischen Aufwand.

Stärker verbreitet sind daher Packungen, bei welchen konventionelle, stückige Katalysatoren eingesetzt werden. Die Katalysatoren werden beispielsweise in Taschen aus Drahtgewebe eingebracht. Hierbei können diese Taschen entweder direkt als Destillationseinbauten dienen, wie es z.B. bei der Bauform KATAPAK-M® der Firma Sulzer AG der Fall ist, oder die ebenen Taschen werden zwischen die einzelnen Schichten der Destillationspackung (z.B. Gewebepackungen zur Stofftrennung) eingelegt, wie es z.B. bei den Packungen der Bauform Multipak® der Firma Montz GmbH der Fall ist. Die Verwendung dieser Packungen ist meist jedoch störanfällig, da die passenden Flüssigkeitsberieselungsdichten genau einzuhalten sind, was sich in der Praxis regelmäßig als schwierig erweist.

Die Verwendung sogenannter "Bales" der Firma CDTech, Houston, USA ist beispielsweise in der EP-A 466954 beschrieben. Auch hier wird der Katalysator in einer Art Taschenstruktur innerhalb der Kolonne fixiert. Dabei ist die Taschenstruktur im Vergleich zur vorgehend geschilderten gröber, was letztlich zu einer Verringerung der erzielbaren Trennstufenzahl je Meter Kolonnenhöhe führt.

Generell stellt sich bei allen Systemen, bei welchen der Katalysator in Taschen eingenäht wird, das Problem, dass ein Entfernen des verbrauchten Katalysators sowie ein Neubefüllen recht umständlich und zeitintensiv ist.

Bei Verwendung von Bodenkolonnen sind verschiedene Unterbringungsmöglichkeiten des Katalysators in Ablaufschächten oder auf dem Boden beschrieben. Eine Sonderform sind suspendierte Katalysatoren in Bodenkolonnen. Hier liegt der Katalysator auf den einzelnen Böden suspendiert vor und wird auf jedem Boden durch Filterelemente zurückgehalten, was konstruktiv und verfahrenstechnisch aufwendig ist. Solche Einbauten sind beispielsweise in der DE 19808385 sowie der US 5,308,451 beschrieben. In dem Verfahren gemäß der US 4,471,145 ist der Katalysator auf den jeweiligen Böden suspendiert und wird hierbei zurückgehalten.

Bei den geschilderten Verfahren gestaltet sich das Entfernen des verbrauchten Katalysators sowie ein Neubefüllen zeitintensiv und verfahrenstechnisch aufwendig. Weiterhin ist es nicht möglich, den Katalysator im laufenden Betrieb zu entfernen und zu ersetzen.

Das Dokument **US-A-5 510 089** beschreibt ein Verfahren zur Durchführung einer Reaktivdestillation in Gegenwart eines heterogenen Katalysators, der als disperse Phase in der Flüssigkeit suspendiert ist, wobei aus der Kolonne bei laufendem Betrieb ein Teil der Katalysator enthaltenden Suspension kontinuierlich abgeführt, in einer Aufbereitungsstufe behandelt und den aufbereiteten Katalysator in die Kolonne zurückgeführt wird.

Es stellte sich somit die Aufgabe, ein Verfahren zu finden, welches die genannten Nachteile vermeidet. Weiterhin soll es dabei den Einsatz herkömmlicher, konstruktiv einfacher Einbauten ermöglichen.

Demgemäss wurde ein Verfahren zur Durchführung einer Reaktivdestillation in Gegenwart eines heterogenen Katalysators, der als disperse Phase in der Flüssigkeit suspendiert ist gefunden, bei welchem man aus der Kolonne einen Teil der Katalysator enthaltenden Suspension kontinuierlich abführt, in einer Aufbereitungsstufe behandelt und den aufbereiteten Katalysator zumindest teilweise in die Kolonne zurückführt.

In einem alternativen Verfahren wird der entnommene Katalysator nicht zurückgeführt und es wird nur bei Bedarf frischer Katalysator zugegeben.

Das erfindungsgemäße Verfahren bietet den Vorteil, dass der Katalysator bei laufendem Betrieb in verfahrenstechnisch einfacher Art und Weise bedarfsgerecht aufbereitet und ggf. zurückgeführt werden kann, wodurch die Wirtschaftlichkeit des Verfahrens gesteigert wird.

Besonders empfiehlt es sich, den Katalysator in Form eines feinteiligen Suspensionskatalysators als disperse Phase in der Flüssigkeit einzusetzen. Als Katalysatoren können alle aus dem Stand der Technik bekannten Katalysatoren eingesetzt werden, die für eine Suspensionsfahrweise geeignet sind. Generell geeignete Katalysatortypen sind zum Beispiel Metall-, Fällungs-, Träger-oder Raney-Typ-Katalysatoren, deren Herstellung beispielsweise in Ullmann, Enzyklopädie der Technischen Chemie, 4. Auflage, 1977, Band 13, Seite 558 bis 665 beschrieben ist. Bei den Nicht-geträgerten Katalysatoren kommen bevorzugt Metall-, besonders bevorzugt Edelmetallkatalysatoren zum Einsatz, wie beispielsweise Platin, Rhodium, Palladium, Cobalt, Nickel, oder Ruthenium. Neben den Metallen der VIII. Nebengruppe des Periodensystems, können aber auch die Metalle der I. und VII. Hauptgruppe, bevorzugt Kupfer und/oder Rhenium eingesetzt werden. Des weiteren können auch Metallsalze und -oxide, wie z.b. Rheniumsulfide, Kupfersulfide, Zinkchromide, Kupferchromide, Nickeloxide, Molybdänoxide, Aluminiumoxide, Rheniumoxide und Zinkoxide eingesetzt werden.

Im Rahmen des erfindungsgemäßen Verfahren können auch sehr vorteilhaft Raney-Typ-Katalysatoren wie zum Beispiel Raney-Nickel, Raney-Kupfer, Raney-Cobalt, Raney-Nickel/Molybdän, Raney-Nickel/Kupfer, Raney-Nickel/Chrom, Raney-Nickel/Chrom/Eisen oder Rhenium-Schwamm eingesetzt werden. Die Herstellung eines Raney-Nickel/Molybdän-Katalysators ist zum Beispiel in US 4,153,578 beschrieben.

Im Rahmen des erfindungsgemäßen Verfahrens werden besonders bevorzugt Trägersuspensionskatalysatoren eingesetzt. Als Trägermaterial können alle bei der Katalysatorherstellung bekannten Trägermaterialien verwendet werden, besonders bevorzugt Aktivkohle, Siliciumcarbid, Aluminiumoxid, Siliciumoxid, Siliciumdioxid, Titandioxid, Zirkoniumoxid, Magnesiumoxid, Zinkoxid, Calciumcarbonat, Bariumsulfat oder deren Gemische. Als Aktivkomponenten können bei den Trägersuspensionskatalysatoren prinzipiell alle Metalle, vorzugsweise Metalle der VIII. Nebengruppe des Periodensystems wie Platin, Rhodium, Palladium, Cobalt, Nickel, Ruthenium oder deren Gemische eingesetzt werden. Ebenfalls vorteilhaft verwendbar sind die Metalle der I., III. und VII. Nebengruppe des Periodensystems, vorzugsweise Kupfer und/oder Rhenium, sowie Yttrium und die Elemente aus der Gruppe der Lanthanoiden, vorzugsweise Lanthan und/oder Praseodym.

Der Gehalt der Aktivkomponente beträgt bei den Trägersuspensionskatalysatoren in der Regel 0,001 bis 30 Gew%, vorzugsweise 0,01 bis 8 Gew%, bezogen auf das Gesamtgewicht des Katalysators.

Die Partikelgröße des Katalysators liegt üblicherweise in einem Bereich von etwa 0,1 bis 500 um, bevorzugt etwa 0,5 bis 200 µm, besonders bevorzugt etwa 1 bis 100 µm Dabei wird durch die mechanische Belastung durch das Pumpen der Suspension die Partikelgröße im Laufe der Zeit verkleinert, bis eine Grenzkorngröße von ca. 1 µm erreicht wird.

Das erfindungsgemäße Verfahren sieht in einer bevorzugten Ausführungsform vor, in der Kolonne Packungen anzuordnen und feinteilige Suspensionskatalysatoren zusammen mit der Flüssigkeit über diese Trenneinbauten fließen zu lassen. Da der Katalysator nicht mehr einer bestimmten Trennstufe zugeordnet ist, sondern über die gesamte gewünschte Kolonnenlänge fließt, muss er nur an einer Stelle, beispielsweise dem Kolonnensumpf oder dem mittleren Kolonnenbereich entnommen werden und beispielsweise durch Filtration abgetrennt und ggf. später wieder zurückgeführt werden. Hierbei können Teilströme ab- und bei Bedarf einer Regeneration zugeführt werden. Die Abtrennung des Katalysators kann sowohl innerhalb als auch außerhalb der Kolonne erfolgen.

Im erfindungsgemäßen Verfahren werden strukturierte Packungen, zum Beispiel aus Drahtgewebe, Blech oder Streckmetall eingesetzt. In einer bevorzugten Ausführungsform wählt man Packungsmaterial und/oder Geometrie der Packung so, dass ein teilweises reversibles Anhaften der Katalysatorteilchen an der Packung erreicht wird. Auswahl ist jeweils von den eingesetzten Stoffen sowie den Rahmenbedingungen abhängig und kann vom Fachmann durch Routineversuche ermittelt werden. Generell ist dabei ähnlich vorzugehen wie bei der Bestimmung des dynamischen Hold-Ups einer Destillationskolonne. Dabei wird im stationären kontinuierlichen Betrieb, der Ablauf und Zulauf der Kolonne gleichzeitig geschlossen. Anschließend wird die Katalysatormenge in der eingeschlossenen Suspension und auf den Einbauten bestimmt. Die so bestimmte Menge an Katalysator pro Reaktionsvolumen sollte dabei größer als die Gesamtmenge an Katalysator bezogen auf den gesamten Flüssigkeitsinhalt des flüssigen Kreislaufstroms sein. Durch das teilweise Anhaften wird vorteilhafterweise die im Kreis zu führende Katalysatormenge begrenzt und durch die Relativbewegung zwischen Katalysatorteilchen und Arbeitslösung eine Beschleunigung der Reaktion erreicht. Beispielsweise eignen sich dafür Drahtgewebepakkungen mit einer hohen spezifischen Oberfläche, wie sie von der Firma Montz mit der Bauart A3 oder der Firma Sulzer mit den Bauarten DX, BX oder EX angeboten werden, die noch zusätzlich aufgeraut wurden. Ebenfalls geeignet sind zusätzlich aufgeraute Blechpackungen, mit oder ohne Perforationen. Bei Verwendung von Perforationen sind diese entsprechend klein zu halten. Beispiele für Blechpackungen sind die Bauarten Montz B1 und BSH sowie die Sulzer Mellapack. Insgesamt sollten die Einbauten Oberflächenrauhigkeiten im Bereich des 0,1- bis 10-fachen, vorzugsweise 0,5-bis 5-fachen, der mittleren Korngröße der suspendierten Katalysatorteilchen aufweisen.

Ein Teilstrom von der den Katalysator enthaltenden Suspension wird bevorzugt im mittleren Bereich der Kolonne oder am bzw. nahe dem Kolonnensumpf entnommen. Die Abtrennung im mittleren Bereich der Kolonne ist immer dann vorteilhaft, wenn es durch hohe Verweilzeiten und hohe Temperaturen im Sumpf zu Nebenreaktionen kommt oder wenn das Wertprodukt von schwersiedenden Komponenten abgereichert oder befreit werden soll. Die Abtrennung des Katalysators von der Flüssigkeit kann durch Trennverfahren wie Filtration, Flotation oder Sedimentation durchgeführt werden. Besonders kann sich die Querstromfiltration empfehlen. Bei Bedarf kann der Katalysator einer allgemein bekannten Regenerierung zugeführt werden und anschließend wieder in die Kolonne geführt werden. Bevorzugt wird der Katalysator im mittleren Bereich der Kolonne oder im oberen Teil der Kolonne zurückgeführt. Vorteilhafterweise können Ausschleusen und Rückführen des Katalysators bei laufendem Betrieb der Kolonne erfolgen. Bevorzugt ist der in die Kolonne zurückgeführte Teilstrom klein, so dass die internen Ströme in der Kolonne bei zurückgeführtem Katalysator nicht mehr als das fünffache, bevorzugt nicht mehr als das zweifache, der internen Ströme ohne Rückführung des Katalysators in die Kolonne beträgt. Darüber hinaus kann auch frischer Katalysator mittels des Kreislaufstroms in die Kolonne gegeben werden.

Bei Verwendung einer Kolonne, die mit in Längsrichtung wirksamen Trenneinrichtungen wie steckbaren Blechen oder einer eingeschweißten Wand ausgestattet ist, können bei Bildung von mehreren Reaktionsprodukten unterschiedlicher Siedereihenfolge gleichzeitig die Reaktionsprodukte in reiner Form erhalten werden.

Es kann sich weiter empfehlen, den im Kreisstrom abgetrennten Katalysator z.b. als Filterkuchen mit möglichst geringer Restfeuchte, erneut zu dispergiert, um eine Rückvermischung mit Edukten zu minimieren.

Bevorzugt eignet sich das erfindungsgemäße Verfahren beispielsweise für Veresterungen, Acetalbildungen, Veretherungen, Aldolisierungen und Hydrierungen. Vorteile sind insbesondere gegeben, wenn zur Steigerung der Raum-Zeit-Ausbeuten große Katalysatorflächen benötigt werden, welche bei groben Katalysatorpartikeln nicht vorliegen.

Ein Beispiel hierfür ist die Aldolisierung von Citral und Aceton zu Pseudojonon.

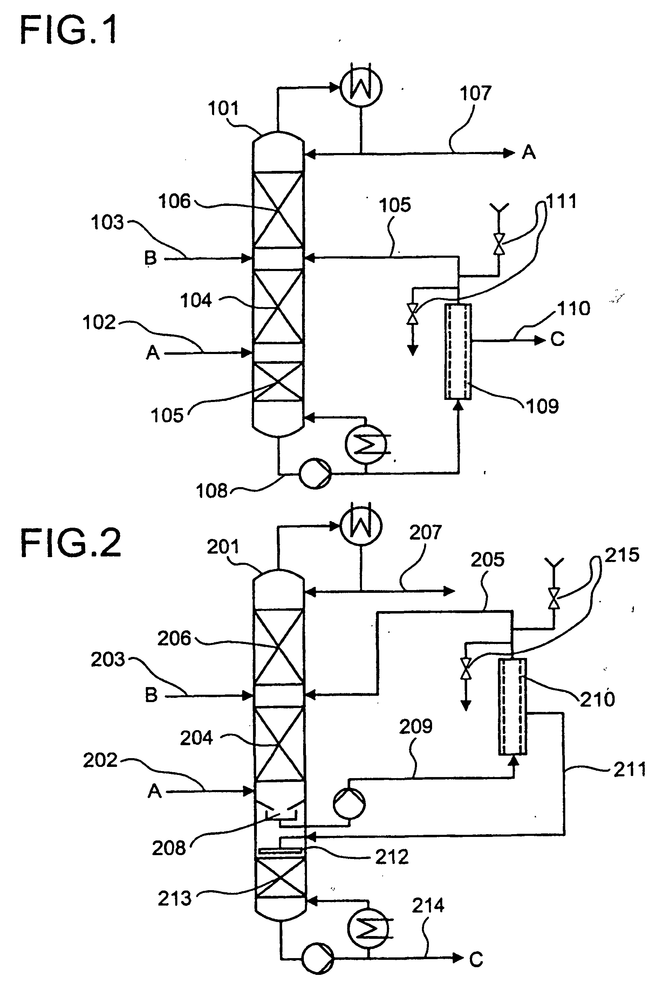

Das erfindungsgemäße Verfahren wird exemplarisch anhand der Figuren 1 bis 3 näher beschrieben.

In Figur 1 ist eine als Reaktionskolonne fungierende Fraktionierkolonne (101) dargestellt, der über die Zuläufe (102) und (103) die Einsatzstoffe A (über 102) und B (über 103) zugegeben werden, welche zum Produkt C weiterreagieren. Hierbei weisen die Stoffe A, B und C ansteigende Siedetemperaturen auf. Innerhalb der Reaktionskolonne sind Einbauten (104) angebracht. Dabei ist es hier von Vorteil, den höher siedenden Reaktanten getrennt oberhalb des tiefer siedenden Reaktanten kontinuierlich in die Reaktionskolonne (im folgenden "Kolonne" genannt) einzuspeisen, da so ein Gegenstrom der Reaktanten bewirkt wird.

Bevorzugt dosiert man den tiefer siedenden Reaktanten gasförmig oder überhitzt in die Kolonne, wenn dadurch eine Spaltung im Sumpf der Kolonne bei hohen Temperaturen und die Bildung von Nebenprodukten vermieden wird.

Der in die Flüssigkeit suspendierte Katalysator wird ebenfalls oberhalb der Einbauten (104) mittels Leitung (105) der Kolonne zugeführt und fließt über die Einbauten. Bevorzugt wird aufgerautes Drahtgewebe eingesetzt, welches ein teilweise reversibles Anhaften der Teilchen an der Packung bewirkt. Hierdurch ist es möglich, in der durch die Einbauten definierten Transportzone eine erhöhte Konzentration an Katalysator zu realisieren. In dieser Zone kommt es zum Kontakt der Einsatzstoffe mit dem Katalysator, so dass es zu der Umsetzung zum Reaktionsprodukt C kommt. Durch das Anhaften des Katalysators an den Einbauten kommt es gleichzeitig vorteilhafterweise zu einer erhöhten Relativgeschwindigkeit zwischen dem Katalysator und der Flüssigkeit, wodurch man den Stoffaustausch begünstigt.

Oberhalb der durch die Einbauten definierten Katalysatortransportzone befindet sich eine Destillationszone (106), in welche destillative Trennelemente eingebracht wurden. Diese Destillationszone sorgt dafür, dass der im oberen Zulauf (103) zugegebene Einsatzstoff B nicht in das Destillat gelangt. Der im Überschuss zudosierte Einsatzstoff A wird als Destillat über Leitung (107) abgetrennt und kann über Leitung (102) erneut der Kolonne im unteren Bereich zugeführt werden.

Unterhalb der Einbauten (104) befindet sich die Zone (105). In dieser Zone wird der in der Flüssigkeit suspendierte Katalysator in den Sumpf der Kolonne transportiert. Gleichzeitig kann diese Zone destillative Aufgaben erfüllen, indem sie das Reaktionsprodukt C von leichtersiedenden Komponenten, insbesondere dem durch Leitung (102) zudosierten Einsatzstoff A, abreichert.

Ein Teilstrom des vom Kolonnensumpf abgezogenen Stroms (108) wird abgezweigt und der suspendierte Katalysator wird bevorzugt durch Querstromfiltration (109) vom Reaktionsprodukt C getrennt. Dabei fällt das Reaktionsprodukt als Permeat (110) an. Der in einem Teilstrom des Reaktionsmediums suspendierte Katalysator wird über Leitung (105) rezirkuliert und oberhalb der Einbauten (104) der Kolonne wieder zugeführt. Mittels Leitung (111) kann je nach Bedarf bei laufendem Betrieb frischer Katalysator zugeführt oder verbrauchter Katalysator entnommen werden.

In Figur 2 ist eine als Reaktionskolonne fungierende Fraktionierkolonne (201) dargestellt, der über die Zuläufe (202) und (203) die Einsatzstoffe A (über 202) und B (über 203) zugegeben werden, welche zum Produkt C weiterreagieren. Hierbei weisen die Stoffe A, B und C ansteigende Siedetemperaturen auf. Innerhalb der Reaktionskolonne sind Einbauten (204) angebracht. Der in die Flüssigkeit suspendierte Katalysator wird ebenfalls oberhalb der Einbauten (204) mittels Leitung (205) der Kolonne zugeführt und fließt über die Einbauten. Die Einbauten ermöglichen eine definierte Transportzone, wodurch man eine erhöhte Konzentration an Katalysator realisieren kann. In dieser Zone kommt es zum Kontakt der Einsatzstoffe mit dem Katalysator, so dass es zu der Umsetzung zum Reaktionsprodukt C kommt. Durch das Anhaften des Katalysators an den Einbauten kommt es gleichzeitig vorteilhafterweise zu einer erhöhten Relativgeschwindigkeit zwischen dem Katalysator und der Flüssigkeit, wodurch man den Stoffaustausch begünstigt.

Oberhalb der durch die Einbauten definierten Katalysatortransportzone befindet sich eine Destillationszone (206), in welche destillative Trennelemente eingebracht wurden. Diese Destillationszone sorgt dafür, dass der im oberen Zulauf (203) zugegebene Einsatzstoff B nicht in das Destillat gelangt. Der im Überschuss zudosierte Einsatzstoff A wird als Destillat über Leitung (207) abgetrennt und kann über Leitung (202) erneut der Kolonne im unteren Bereich zugeführt werden. Im Flüssigkeitssammler (208), der direkt unterhalb der Einbauten (204) angeordnet ist, wird die Flüssigkeit gesammelt und über Leitung (209) der Filtration (210) zugeführt. Das Permeat (211) wird in einem Verteiler (212) oberhalb der Zone (213) wieder aufgegeben. Diese Zone (213) erfüllt destillative Aufgaben, indem sie das Reaktionsprodukt C von leichtersiedenden Komponenten, insbesondere dem durch Leitung (202) zudosierten Einsatzstoff A, abreichert. Das Reaktionsprodukt C fällt als Sumpfstrom (214) der Kolonne an.

Der suspendierte Katalysator wird bevorzugt durch Querstromfiltration (210) vom Reaktionsprodukt C getrennt. Der in einem Teilstrom des Reaktionsmediums suspendierte Katalysator wird über Leitung (205) rezirkuliert und oberhalb der Einbauten (104) der Kolonne wieder zugeführt. Mittels Leitung (215) kann je nach Bedarf bei laufendem Betrieb frischer Katalysator zugeführt oder verbrauchter Katalysator entnommen werden.

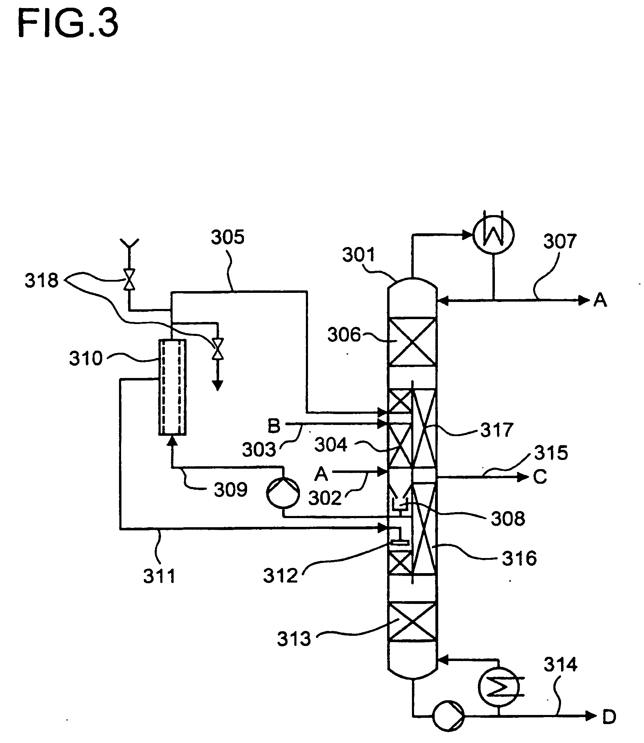

In Figur 3 ist eine als Reaktionskolonne fungierende Fraktionierkolonne (301) dargestellt, der über die Zuläufe (302) und (303) die Einsatzstoffe A (über 302) und B (über 303) zugegeben werden, welche zu den Produkten C und D weiterreagieren. Hierbei weisen die Stoffe A, B, C und D ansteigende Siedetemperaturen auf. Die Kolonne wird durch eine in Längsrichtung wirksame Trenneinrichtung unterhalb und oberhalb der Zulaufstellen für die Einsatzstoffe A und B unterteilt. Der in der Flüssigkeit suspendierte Katalysator wird wie der Einsatzstoff B ebenfalls oberhalb der Einbauten (304) mittels Leitung (305) der Kolonne zugeführt und fließt über die Einbauten. Die Einbauten ermöglichen eine definierte Transportzone, wodurch man eine erhöhte Konzentration an Katalysator realisieren kann. In dieser Zone kommt es zum Kontakt der Einsatzstoffe mit dem Katalysator, so dass es zu der Umsetzung zu den Reaktionsprodukten C und D kommt. Durch das Anhaften des Katalysators an den Einbauten kommt es gleichzeitig vorteilhafterweise zu einer erhöhten Relativgeschwindigkeit zwischen dem Katalysator und der Flüssigkeit, wodurch man den Stoffaustausch begünstigt.

Oberhalb der durch die Einbauten definierten Katalysatortransportzone befindet sich eine Destillationszone (306), in welche destillative Trennelemente eingebracht wurden. Diese Destillationszone sorgt dafür, dass das Reaktionsprodukt C nicht in das Destillat gelangt. Der im Überschuss zudosierte Einsatzstoff A wird als Destillat über Leitung (307) abgetrennt und kann über Leitung (302) erneut der Kolonne im unteren Bereich zugeführt werden. Im Flüssigkeitssammler (308), der direkt unterhalb der Einbauten (304) angeordnet ist, wird die Flüssigkeit gesammelt und über Leitung (309) der Filtration (310) zugeführt. Das Permeat (311) wird in einem Verteiler (312) oberhalb der Zone (313) wieder aufgegeben. Das Reaktionsprodukt D fällt gemäß der Siedereihenfolge als Sumpfstrom (314) an. Die Zone (313) erfüllt destillative Aufgaben, indem sie das Reaktionsprodukt D von leichtersiedenden Komponenten, insbesondere von dem leichtersiedenden Reaktionsprodukt C, abreichert. Das Reaktionsprodukt C wiederum kann über Leitung (315) in sehr reiner Form abgezogen werden. Dies wird durch die in Längsrichtung wirksame Trenneinrichtung und den Destillationszonen (316) und (317) erreicht. Das Reaktionsprodukt gelangt aufgrund seiner Siedereihenfolge, die zwischen dem Edukt A und dem Reaktionsprodukt D liegt, sowohl in die Destillationszone (316) als auch in die Destillationszone (317). Während in der Destillationszone (316) das Reaktionsprodukt C von schwersiedenden Komponenten, insbesondere dem Reaktionsprodukt D, abgereichert wird, erfolgt in der Destillationszone (317) eine Abtrennung von leichtersiedenden Komponenten, insbesondere dem Edukt A aus dem Produktstrom der Reaktion. Die in Längsrichtung wirksame Trenneinrichtung verhindert eine Quervermischung der Flüssigkeits- und Brüdenströme aus der Zone (304) mit den Flüssigkeits- und Brüdenströmen aus den Zonen (316) und (317).

Der suspendierte Katalysator wird bevorzugt durch Querstromfiltration (310) vom Reaktionsprodukt C getrennt. Der in einem Teilstrom des Reaktionsmediums suspendierte Katalysator wird über Leitung (305) rezirkuliert und oberhalb der Einbauten (304) der Kolonne wieder zugeführt. Der Katalysator wird vorteilhafterweise nur im Bereich der Einbauten (304) durch die Kolonne transportiert. Mittels Leitung (318) kann je nach Bedarf bei laufendem Betrieb frischer Katalysator zugeführt oder verbrauchter Katalysator entnommen werden.

### Beispiel

### Aldolkondensation von Aceton und Citral zu Pseudojonon

Die Umsetzung von Aceton mit Citral erfolgte in einer Versuchskolonne, die schematisch Figur 2 entsprach. Die Kolonne hatte einem Durchmesser von 0,055 m und wurde im oberen Bereich (206) und im unteren Bereich (213) über eine Höhe von jeweils 0,6 m mit einer Drahtgewebepackung des Typs A3-500 der Firma Montz GmbH, Hilden bestückt. Der Bereich (204) hingegen wurden über eine Höhe von 0,6 m mit einer aufgerauten Drahtgewebepackung des Typs A3-1200 der Firma Montz GmbH, Hilden bestückt. Über dem Zulauf (203) wurde kontinuierlich 55 g/h Citral und über den Zulauf (202) kontinuierlich 210 g/h Aceton der Kolonne zugeführt. Am Kopf der Kolonne wurde 196 g/h Destillat bestehend aus 96,2% Aceton, 0,4% Diacetonalkohol, 0,2% Mesityloxid und 3,2% Wasser abgezogen.

Die Zufuhr der Katalysatorsuspension erfolgte gemeinsam mit dem rezirkulierten Produktstrom über den Zulauf (205). Als Suspensionskatalysator wurde ein mit Praseodym beschichteter Aluminiumoxid-Katalysator in Pulverform verwendet. Der Praseodymgehalt betrug 5 Gew%. Die über den Zulauf (205) zugeführte Suspension hatte einen auf die Masse bezogenen Feststoffanteil von 20%.

Im Flüssigkeitssammler (208), der direkt unterhalb der Einbauten (204) angeordnet war, wurde die Flüssigkeit gesammelt und über Leitung (209) der Querstromfiltration (210) zugeführt. Die Flüssigkeit enthielt dabei ca. 64,2 Gew% Aceton, 0,2 Gew% Wasser, 0,1 Gew% Mesityloxid, 0,3% Diacetonalkohol, 31,5 Gew% Pseudojonon, 1,3% Citral und 0,5% Hochsieder. Der auf die Masse bezogene Feststoffanteil betrug ca. 5%. Zur Filtration wurde eine Filtereinheit ähnlich der kommerziell erhältlichen Filtermodule der Firma Membraflow, Aalen-Essingen verwendet.

Das Permeat (211) wurde in einem Verteiler (212) oberhalb der Zone (213) wieder aufgegeben. Als Sumpfstrom der Kolonne wurde 69 g/h Rohprodukt bestehend aus ca. 94,4 % Pseudojonon, 4,0 % Citral und 1,6 % Hochsieder entnommen.

## Patentansprüche

1. Verfahren zur Durchführung einer Reaktivdestillation in Gegenwart eines heterogenen Katalysators, der als disperse Phase in der Flüssigkeit suspendiert ist, wobei man aus der Kolonne
• einen Teil der Katalysator enthaltenden Suspension kontinuierlich abführt,
• in einer Aufbereitungsstufe behandelt und
• den aufbereiteten Katalysator zumindest teilweise in die Kolonne zurückführt, **dadurch gekennzeichnet, dass** man in dem Bereich, in dem sich der Katalysator in der Kolonne befindet, strukturierte Packungen als Einbauten einsetzt.

2. Verfahren nach Anspruch 1, **dadurch gekennzeichnet, dass** man den Katalysator bei laufendem Betrieb der Kolonne abführt.

3. Verfahren nach Ansprüchen 1 oder 2, **dadurch gekennzeichnet, dass** man den aufbereiteten und/oder frischen Katalysator bei laufendem Betrieb in die Kolonne führt.

4. Verfahren nach Anspruch 1, **dadurch gekennzeichnet, dass** man Packungen einsetzt, bei welchen die Katalysatorteilchen teilweise reversibel anhaften.

5. Verfahren nach Anspruch 4, **dadurch gekennzeichnet, dass** man Drahtgewebe als Packungen einsetzt.

6. Verfahren nach Anspruch 5, **dadurch gekennzeichnet, dass** aufgerautes Drahtgewebe einsetzt.

7. Verfahren nach Ansprüchen 1 bis 6, **dadurch gekennzeichnet, dass** man den Katalysator im mittleren Bereich der Kolonne abtrennt und später wieder zuführt.

8. Verfahren nach Ansprüchen 1 bis 6, **dadurch gekennzeichnet, dass** man den Katalysator im Sumpf der Kolonne abtrennt und später wieder zuführt.

9. Verfahren nach Ansprüchen 1 bis 8, **dadurch gekennzeichnet, dass** man in der Kolonne in Längsrichtung wirksame Trenneinrichtungen einsetzt.

10. Verfahren nach Ansprüchen 1 bis 9, **dadurch gekennzeichnet, dass** man den Katalysator durch Filtration, Flotation oder Sedimentation abtrennt.

11. Verfahren nach Anspruch 10, **dadurch gekennzeichnet, dass** man eine Querstromfiltration als Filtration durchführt.

12. Verfahren nach Ansprüchen 1 bis 10, **dadurch gekennzeichnet, dass** man den abgetrennten Katalysator durch eine Wäsche mit einer Lauge, Base oder einem Lösungsmittel, durch Kalzination oder einem anderen Verfahren regeneriert.

13. Verwendung der Verfahren gem. Ansprüchen 1 bis 12 für Veresterungen, Acetalbildungen, Veretherungen, Aldolisierungen oder Hydrierungen.

14. Verwendung des Verfahren gem. Ansprüchen 1 bis 12 für die Aldolisierung von Aceton und Citral zu Pseudojonon.

## Claims

1. A process for carrying out a reactive distillation in the presence of a heterogeneous catalyst which is suspended as disperse phase in the liquid, wherein
• part of the catalyst-containing suspension is continuously discharged from the column and
• is treated in a work-up stage and
• at least part of the worked-up catalyst is returned to the column, wherein structured packing is used as internals in the region in which the catalyst is located in the column.

2. The process according to claim 1, wherein the catalyst is discharged from the column during operation of the column.

3. The process according to claim 1 or 2, wherein the worked-up and/or fresh catalyst is fed into the column during operation of the column.

4. The process according to claim 1, wherein some of the catalyst particles adhere reversibly to the packing used.

5. The process according to claim 4, wherein wire mesh is used as packing.

6. The process according to claim 5, wherein roughened wire mesh is used.

7. The process according to any of claims 1 to 6, wherein the catalyst is separated off in the middle region of the column and is later fed back in.

8. The process according to any of claims 1 to 6, wherein the catalyst is separated off at the bottom of the column and is later fed back in.

9. The process according to any of claims 1 to 8, wherein separation devices which are effective in the longitudinal direction are installed in the column.

10. The process according to any of claims 1 to 9, wherein the catalyst is separated off by filtration, flotation or sedimentation.

11. The process according to claim 10, wherein a crossflow filtration is carried out as filtration.

12. The process according to any of claims 1 to 10, wherein the catalyst which has been separated off is regenerated by washing with a caustic alkali, a base or a solvent, by calcination or by another method.

13. The use of a process according to any of claims 1 to 12 for esterifications, acetal formations, etherifications, aldolizations or hydrogenations.

14. The use of a process according to any of claims 1 to 12 for the aldolization of acetone and citral to form pseudoionone.

## Revendications

1. Procédé de mise en oeuvre d'une distillation réactive en présence d'un catalyseur hétérogène, qui est en suspension dans le liquide sous la forme d'une phase dispersée, dans lequel, à partir de la colonne,
- on évacue en continu une partie de la suspension contenant du catalyseur,
- on la traite dans une étape de traitement, et
- on recycle au moins partiellement dans la colonne le catalyseur traité,
**caractérisé en ce que**, dans la zone dans laquelle le catalyseur se trouve dans la colonne, on met en oeuvre des garnissages structurés comme éléments encastrés.

2. Procédé suivant la revendication 1, **caractérisé en ce qu'**on évacue le catalyseur au cours d'une mise en service continue de la colonne.

3. Procédé suivant les revendications 1 ou 2, **caractérisé en ce que**, lors d'une mise en service continue, on conduit dans la colonne le catalyseur traité et/ou frais.

4. Procédé suivant la revendication 1, **caractérisé en ce qu'**on met en oeuvre des garnissages auxquels les particules de catalyseur adhèrent de manière partiellement réversible.

5. Procédé suivant la revendication 4, **caractérisé en ce que**, comme garnissages, on met en oeuvre des tissus métalliques.

6. Procédé suivant la revendication 5, **caractérisé en ce qu'**on met en oeuvre des tissus métalliques rendus rugueux.

7. Procédé suivant les revendications 1 à 6, **caractérisé en ce qu'**on isole le catalyseur dans la zone centrale de la colonne et on le ramène plus tard.

8. Procédé suivant les revendications 1 à 6, **caractérisé en ce qu'**on isole le catalyseur dans le fond de la colonne et on le ramène plus tard.

9. Procédé suivant les revendications 1 à 8, **caractérisé en ce que**, dans la colonne, on met en oeuvre des dispositifs séparateurs efficaces en direction longitudinale.

10. Procédé suivant les revendications 1 à 9, **caractérisé en ce qu'**on isole le catalyseur par filtration, flottation ou sédimentation.

11. Procédé suivant la revendication 10, **caractérisé en ce que**, comme filtration, on effectue une filtration en courant transversal.

12. Procédé suivant les revendications 1 à 10, **caractérisé en ce qu'**on régénère le catalyseur isolé par un lavage avec une lessive, une base ou un solvant, par calcination ou un autre procédé.

13. Utilisation des procédés suivant les revendications 1 à 12, pour des estérifications, des formations d'acétal, des éthérifications, des aldolisations ou des hydrogénations.

14. Utilisation du procédé suivant les revendications 1 à 12, pour l'aldolisation d'acétone et de citral en pseudo-ionone.
